# EUROPÄISCHE PATENTSCHRIFT

(11) **EP 1 802 236 B1**
(45) Veröffentlichungstag und Bekanntmachung des Hinweises auf die Patenterteilung: **16.04.2008**
(21) Anmeldenummer: 05791408.7
(22) Anmeldetag: 18.10.2005
(51) Int. Cl.: A61B 17/00, A61B 17/08

(54) **AUFKLEBBARER GEFÄSSVERSCHLUSS**
ADHESIVE VESSEL CLOSURE
SYSTEME D'OBTURATION DE VAISSEAU SANGUIN ADHESIF

(30) Priorität: 20.10.2004 CH 17302004
(43) Veröffentlichungstag der Anmeldung: 04.07.2007
(73) Patentinhaber: VOSTRA-MED AG, 6330 Cham (CH)
(72) Erfinder: HARREN, Ernst-Diethelm, CH-6312 Steinhausen (CH); LEHMANN, Marc, Ulrich, CH-8704 Herrliberg (CH)
(74) Vertreter: EGLI-EUROPEAN PATENT ATTORNEYS
(86) Internationale Anmeldenummer: PCT/CH2005/000608
(87) Internationale Veröffentlichungsnummer: WO 2006/042430

(56) Entgegenhaltungen:
- DE-A- 4 429 230

## Beschreibung

Die Erfindung betrifft einen aufklebbaren Gefässverschluss nach Patentanspruch 1. Er ist zur Verwendung am menschlichen oder tierischen Körper vorgesehen.

Die Erfindung betrifft insbesondere einen ortsdefiniert aufklebbaren Gefässverschluss mit einem Druckkörper, der geeignet ist, mit einer Wunde und/oder Hautverletzung in Berührung zu gelangen.

In diesem Bereich der medizinischen Anwendungen, insbesondere aber bei der Punktierung und/oder unfallbedingter ungewollter Eröffnung von Gefässen, gibt es noch immer einen Bedarf, einen auch für einen medizinischen Laien einfach zu handhabenden Gefässverschluss zur Verfügung zu stellen, mit dem nicht nur der anschliessende Blutaustritt bzw. Blutverlust in den freien Raum ausserhalb des Körpers, sondern auch der Blutaustritt in das Gewebe (Hämatombildung) verhindert wird, wobei gleichzeitig aber auch die lebenswichtige Blutzirkulation innerhalb des Körpers nach erfolgtem Einsatz und endgültigem Anlegen des aufklebbaren Gefässverschlusses nicht behindert werden soll.

In herkömmlicher Weise geschieht das Verschliessen von Punktionswunden eines Blutgefässes so, dass nach dem Eingriff an der Gefässeinstichstelle im richtigen Mass Druck ausgeübt wird. Wegen der Gefahr der Hämatombildung darf der Druck nicht zu klein sein, wegen der Gefahr der Kreislaufunterbindung im betroffenen Körperteil aber auch nicht zu gross. Es gibt deshalb Lösungen, bei denen versucht wird, einen adäquaten Gegendruck, also einen dem Blutdruck entsprechenden Gegendruck, mittels einer durch das austretende Blut sich füllenden externen Blutkammer bzw. externen Druckkammer und in dieser Weise als Druckkörper wirkenden Vorrichtung, aufzubauen. Zur Unterstützung werden teilweise noch zusätzliche mechanische Druckkörper eingesetzt.

Ein Beispiel einer derartigen Vorrichtung ist in der DE-44 29 230 beschrieben. Sie zeigt einen aufklebbaren Punktionsverschluss mit einer Druckkammer und einer dehnbaren Druckwand. Dieser Punktionsverschluss wird bereits vor dem Punktieren der Arterie aufgeklebt. Beim Punktieren wird die Druckwand, je nach Ausführungsart aber auch das darüber liegende Halteband, durchstochen. Bei den Ausführungsarten bei denen das darüber liegende Halteband nicht durchstochen wird, sind zusätzliche unterstützende Druckkörper, entweder Druckpolster oder luftgefüllte Plastikkugeln, vorhanden. Dabei werden diese zusätzlichen unterstützenden Druckkörper auf das Halteband aufgebracht und ragen durch eine Öffnung des Haltebandes in die erwähnte Druckkammer. Die Unterseite des Haltebandes ist mit Klebstoff versehen.

Der aufklebbare Punktionsverschluss gemäss DE-44 29 230 hat allerdings den Nachteil, dass er im Aufbau, wegen der stets vorhandenen Druckkammer, relativ kompliziert ist. Zudem ist er in der Handhabung nicht ganz einfach, zum Einen, weil die zu punktierende Arterie stets durch die dehnbare Druckwand der Druckkammer punktiert werden muss, zum Anderen, weil die Teile mit den unterstützenden Druckkörpern nach der Punktierung im wundnahen Bereich noch verklebt werden müssen und die Gefahr besteht, dass vorher noch austretendes Blut auf die vorgesehenen Klebestellen gelangt.

Aufgabe der Erfindung ist es deshalb, einen verbesserten aufklebbaren Gefässverschluss anzugeben.

Diese Aufgabe wird durch die Merkmale des Patentanspruchs 1 gelöst.

Die Lösung besteht darin, dass bei dem aufklebbaren Gefässverschluss eine in der Regel dünne, folienartige Trägerschicht vorhanden ist. Auf dieser Trägerschicht ist verschlussseitig mittels einer technischen Kleberschicht ein Verschlusselement aufgebracht, wobei das Verschlusselement eine Verschlussschicht und einen Druckkörper aufweist und wobei die Verschlussschicht und der Druckkörper einstückig ausgeführt sind.

Durch die Einführung einer Trägerschicht und einer darauf mittels einer Kleberschicht angebrachten Verschlussschicht wird erreicht, dass für die Verschlussschicht, die hier einstückig mit einem zugehörigen Druckkörper ausgebildet ist, eine Vielzahl von geeigneten Materialien verwendbar werden, ohne dass man gleichzeitig wegen fehlenden passenden und geeigneten Hautkleberschichten an deren Einsatz gehindert ist. Die Trägerschicht dient somit einer gewissen ,Entkopplung' der Funktionen und sie dient auch einer Stabilisierung der mechanischen Eigenschaften der Verschlussschicht, z.B. der Dehnbarkeit, die bei gewissen Materialien mit sehr hohen Dehnfähigkeiten, wie z.B. Silikon, notwendig und erwünscht ist. Vorzugsweise wird als Material für den Druckkörper und die Verschlussschicht wegen der haut- und körperfreundlichen Eigenschaften denn auch Silikon eingesetzt.

Somit wird der Aufbau des ortsdefiniert aufklebbaren Gefässverschlusses wesentlich einfacher, was die Herstellungskosten senkt. Auch die Handhabung wird einfacher und sicherer. Dies gilt im Besonderen auch bei unfallbedingten Gefässverletzungen zur Erstversorgung durch medizinischen Laien.

Schliesslich hat der erfindungsgemässe ortdefiniert aufklebbare Gefässverschluss auch unter hygienischen Aspekten Vorteile gegenüber bekannten und gängigen Lösungen. Er ist als steriles Einwegprodukt vorgesehen um insbesondere auch Kreuzinfektionen auszuschliessen.

Das Material für die Trägerschicht wird vorzugsweise so ausgewählt, dass es eine gewisse, allerdings geringere, Dehnfähigkeit als die Verschlussschicht besitzt. Damit wird die beispielsweise bei Silikon vorhandene ausserordentlich hohe Dehnfähigkeit in der flächigen Ausdehnbarkeit begrenzt und stabilisiert. Dies ist meist notwendig um den zur Blutstillung notwendigen Gegendruck aufbringen zu können.

Wie bereits erwähnt ist Silikon, wegen der hervorragenden medizinischen Eignung, eines der bevorzugten Materialien für den Druckkörper. Silikon ist andererseits aber auch ein Material, das nicht problemlos mit allen anderen Materialien verklebbar ist. Insbesondere das zuverlässige direkte und dauerhafte Aufbringen eines technischen Klebers bzw. eines medizinischen Hautklebers, der zusätzlich auch noch die gewünschten Eigenschaften bezüglich Hautverträglichkeit aufweist, ist bisher noch nicht zufriedenstellend gelöst. Deshalb ist die vorhandene Trägerschicht in doppelter Hinsicht nützlich. Der erfindungsgemäss zwischen der Verschlussschicht und der Trägerschicht verwendete Kleber braucht keine hautkleberspezifischen Eigenschaften aufzuweisen.

Es kann auch vorgesehen sein, dass die Kleberschicht, die im einfachsten Fall nur aus einem Kleber (wie oben erwähnt auch als technischer Kleber bezeichnet, d.h. ein Kleber ohne hautkleberspezifische Eigenschaften) besteht, einen Trägerfilm aufweist, der beidseitig mit Kleber versehen ist. Ein solcher Aufbau der Kleberschicht kann gewählt werden, um die Produktion in dem Sinne zu vereinfachen, dass die Kleberschicht auch als 'Zulieferteil' für die Herstellung von aufklebbaren Gefässverschlüssen konzipiert werden kann.

Um den dauerhaften Kontakt von Silikon mit dem verwendeten Kleber zu verbessern kann auch eine Oberflächenbehandlung wie z.B. eine Plasmabehandlung, eine Coronabehandlung, eine nasschemische oder eine sonstige Behandlung vorgesehen sein.

Selbstverständlich können für den Druckkörper auch andere Materialien mit medizinisch günstigen Eigenschaften eingesetzt werden. In Frage kommen beispielsweise Naturkautschuk, Kunstkautschuk, Gummi, Latex, Hydrogel, polymerem Kunststoff, etc. Ebenso sind Kombinationen dieser Materialien, auch mit Silikon, einsetzbar. Auch bei vielen anderen Materialien ausser Silikon erweist sich das Vorhandensein einer Trägerschicht als hilfreich um bei der (nach medizinischen Kriterien zu erfolgenden) Auswahl nicht auch noch Einschränkungen hinsichtlich der Kleberwahl hinnehmen zu müssen.

Der erfindungsgemässe ortsdefiniert aufklebbare Gefässverschluss ist zudem vorzugsweise mit geeignet langen Haltebändern versehen. Neben der guten Handhabbarkeit soll damit natürlich auch eine genügende Haftung auf der Haut erreicht werden, um den zur Blutstillung nötigen Gegendruck aufbauen zu können. Selbstverständlich ist die Geometrie des ortsdefiniert aufklebbaren Gefässverschlusses aber nicht an eine solche Form gebunden. Andere Formen, wie beispielsweise grössere kreisförmige Patches sind ebenso denkbar.

Im folgenden wird der erfindungsgemässe ortsdefiniert aufklebbare Gefässverschluss anhand eines Ausführungsbeispieles mit Zeichnungen detailliert beschrieben.

In den Zeichnungen zeigen:
- Fig. 1: Einen Längsschnitt durch einen ortsdefiniert aufklebbaren Gefässverschluss,
- Fig. 2: Eine Ansicht von unten auf einen Gefässverschluss gemäss Fig. 1, und
- Fig. 3: Eine schematische Skizze zur Anwendung des erfindungsgemässen Gefässverschlusses.

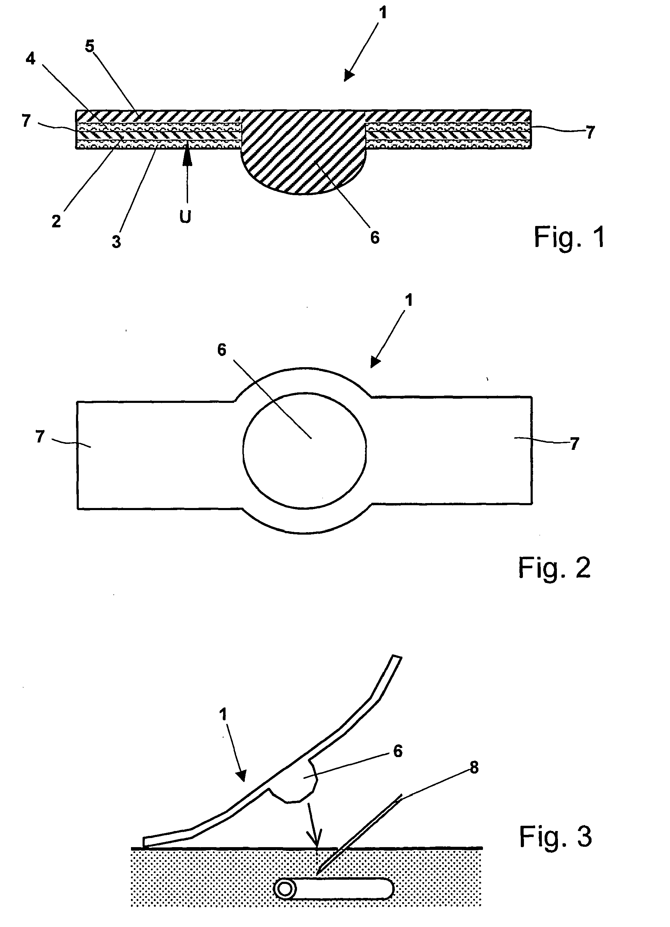

Die Figur 1 zeigt einen nicht-massstäblichen Längsschnitt durch einen ortsdefiniert aufklebbaren Gefässverschluss 1. Eine in der Regel dünne, folienartig ausgebildete Trägerschicht 2 ist beidseitig mit Klebstoffschichten versehen. An einer Unterseite U der Trägerschicht 2 befindet sich eine (medizinische) Hautkleberschicht 3, wobei diese Seite des aufklebbaren Gefässverschlusses 1 zum Aufkleben auf die menschliche Haut vorgesehen ist. Auf der gegenüberliegenden Seite der Trägerschicht 2 befindet sich eine (technische) Kleberschicht 4. Die Kleberschicht 4 befindet sich zwischen der Trägerschicht 2 und einer Verschlussschicht 5 und hat keine hautkleberspezifischen Eigenschaften. Da für die Verschlussschicht 5 Materialien wie beispielsweise Silikon verwendet werden, für die nur Kleber bekannt sind die keine oder keine besonders gute Hautverträglichkeit aufweisen, braucht bei diesem Aufbau des ortsdefiniert aufklebbaren Gefässverschlusses 1 die Kleberschicht 4 keine hautkleberspezifischen bzw. besonders hautfreundlichen Eigenschaften aufzuweisen, da sie keinen Hautkontakt hat.

Die Trägerschicht (2), die Verschlussschicht (5), die Kleberschicht (4) und die Hautkleberschicht (3) sind vorzugsweise alle transparent oder annähernd transparent um das Aufbringen des ortsdefiniert aufklebbaren Gefässverschlusses möglichst zu erleichtern.

Die Verschlussschicht 5 und ein Druckkörper 6 , vorzugsweise ein etwa linsenförmiges Gebilde, sind einstückig ausgeführt. Dabei ragt der Druckkörper 6 durch eine Öffnung in der Trägerschicht 2.

Wie insbesondere aus der Fig. 2 hervorgeht, hat eine bevorzugte Ausführungsform der Erfindung zu beiden Seiten des Druckkörpers 6 Haltebänder 7. Diese können wasserdampfdurchlässig ausgeführt sein.

Die Trägerschicht 2 ist vorzugsweise aus einem Material, das weniger dehnbar ist als die Verschlussschicht 5. Damit wird die höhere Dehnfähigkeit der Verschlussschicht 5 in der flächigen Ausdehnung begrenzt und stabilisiert. Wird für die Kleberschicht 4 ein Schichtaufbau mit einem Trägerfilm und beidseitig auf dem Trägerfilm aufgebrachtem (technischen) Kleber verwendet, so ist natürlich zu erwarten, dass dieser Trägerfilm, ebenso wie die Trägerschicht 2, und natürlich auch in Abhängigkeit von der Materialwahl, zur Ausdehnungsbegrenzung und Stabilisierung der Verschlusschicht 5 beiträgt.

In einer möglichen Ausführungsform besteht die Trägerschicht 2 aus Polyurethan, ist sehr dünn und weist eine Dicke im Bereich von 0.01 bis 0.05 mm auf. Es können aber auch andere Materialien wie Polyethylen verwendet werden.

Die Trägerschicht 2 kann auch flüssigkeitsabsorbierende Eigenschaften aufweisen. Dazu können beispielsweise Materialien wie Vliessstoff oder Papier eingesetzt werden.

Die Verschlussschicht 5 zusammen mit dem Druckkörper 6 besteht vorzugsweise aus Silikon und weist eine Dicke im Bereich von 3 bis 30 mm auf. Wegen der ausserordentlichen Dehnbarkeit weist Silikon auch die erwünschten hohen Rückstellkräfte auf. Es können aber auch andere Materialien wie Naturkautschuk, Kunstkautschuk, Gummi, Latex, Hydrogel, polymerer Kunststoff oder einer Kombination dieser Materialien verwendet werden. Der Druckkörper 6 hat im Normalfall (bei einer Untersuchung etc.) direkten Hautkontakt. Im Falle einer unfallbedingten ungewollten Gefässeröffnung kann der Druckkörper 6 jedoch auch direkten Wundkontakt haben. Deshalb sind für die verwendeten Materialien natürlich geeignete medizinische Eigenschaften, vor allem hinsichtlich der Haut- und Körperverträglichkeit, erforderlich. Die Verschlussschicht, bzw. die Haltebänder sind dabei 1 - 4 mm dick, das linsenförmige Gebilde des Druckkörpers 6 mindestens 3 - 30 mm dick.

Wird Silikon als Verschlussschicht 5 und als Druckkörper 6 verwendet, so kann es notwendig sein, die Kontaktfläche mit der Kleberschicht 4 in geeigneter Weise vorzubereiten. Um den dauerhaften Kontakt von Silikon mit dem verwendeten Kleber zu verbessern kann deshalb eine Oberflächenbehandlung wie z.B. eine Plasma-, Corona-, nasschemische- oder sonstige Behandlung vorgesehen sein.

Schliesslich muss der ortsdefiniert aufklebbare Gefässverschluss 1 hautkleberseitig zusätzlich noch mit abziehbaren Schutzfolien versehen sein (nicht dargestellt). Dabei genügt es in der Regel, dass lediglich die Hautkleberschicht 3 von den abziehbaren Schutzfolien bedeckt ist.

Zur Verwendung des ortsdefiniert aufklebbaren Gefässverschlusses 1 im Falle der Punktierung eines Gefässes: (Vorbemerkung: Die Platzierung, bzw. das ortsdefinierte Anbringen derartiger Gefässverschlüsse wird oft falsch ausgeführt; insofern nämlich, als die Punktionswunde in der Haut von der Gefässpunktionswunde auf Grund des diagonalen Einstichwinkels meist mehrere Millimeter entfernt ist, und dies oft nicht beachtet wird.)
- Das zur Punktierung vorgesehene Blutgefäss, in der Regel eine Arterie, in besonderen Fällen aber auch eine Vene, wird lokalisiert. Da mit Kanülen in der Regel diagonal punktiert wird, ist bekannt, dass die Einstichstelle in der Haut in der Regel einige Millimeter von der Gefässeinstichstelle entfernt liegt.
- Die zu punktierende Körperstelle wird in herkömmlicher Weise gereinigt und desinfiziert.
- Beim ortsdefiniert aufklebbaren Gefässverschluss 1 werden die Schutzfolien (in den Figuren nicht dargestellt), abgezogen und der Gefässverschluss 1 kann mit der Unterseite U der Trägerschicht 2 auf die betreffende Körperstelle (neben der Einsstichstelle) einseitig mit einem der Haltebänder so fixiert werden, dass der Druckkörper 6 später bei der vollständigen ortsdefinierten Applikation des Gefässverschlusses 1 möglichst präzis über die Gefässeinstichstelle (und nicht über die Hauteinstichstelle) zu liegen kommt (vgl. dazu Fig. 3). In diesem Sinne ist die "ortsdefierte Aufklebbarkeit" in diesem Text zu verstehen.
- Die Punktierung wird vorgenommen. Dem zu behandelnden Patienten wird die vorgesehene Menge Flüssigkeit durch die Nadel oder Kanüle entnommen oder zugeführt.
- Nach erfolgtem Eingriff wird die Nadel oder Kanüle 8 herausgezogen. Um Blutungen und Blutverlust möglichst zu vermeiden oder gering zu halten, wird der Druckkörper 6 nun möglichst rasch auf den Ort der Gefässeinstichstelle appliziert und mit dem zweiten Halteband fest auf der Haut aufgeklebt. In besonders geeigneten Fällen kann es erwünscht sein, dass die Hauteinstichstelle und die Gefässeinstichstelle gemeinsam vom Druckkörper 6 abgedeckt werden.

Zur Verwendung des ortsdefiniert aufklebbaren Gefässverschlusses 1 im Falle von unfallbedingten, ungewollten Gefässeröffnungen:
- In der Regel muss zunächst ein Stauverband angelegt werden, um das Ausströmen von Blut aus der Wunde zu stoppen und um die Umgebung der Wunde für das Anlegen des ortsdefiniert aufklebbaren Gefässverschlusses 1 vorbereiten zu können.
- Die Umgebung der Wunde wird gesäubert und die Hautbereiche, auf denen die Haltebänder 7 des ortsdefiniert aufklebbaren Gefässverschlusses 1 angebracht werden, müssen getrocknet werden.
- Der ortsdefiniert aufklebbaren Gefässverschlusses 1 wird in der oben beschriebenen Weise angelegt.
- Der Stauverband wird zunächst gelockert, um das korrekte Anliegen des ortsdefiniert aufklebbaren Gefässverschlusses 1 zu überprüfen. Wenn dies der Fall ist, kann der Stauverband ganz entfernt werden.

Um in Notsituationen bei unfallbedingten, ungewollten Gefässeröffnungen rasche und wirksame Hilfe leisten zu können, kann auch vorgesehen sein, den erfindungsgemässen ortsdefiniert aufklebbaren Gefässverschluss 1 mit so langen Haltebändern 7 zu versehen, dass dieselben beispielsweise vollständig um die betroffenen Gliedmassen oder Körperteile gelegt werden können, um unmittelbar nach dem Anlegen zunächst die Wirkung eines Stauverbandes zu erzielen. Eine derartige vollständige Umschlingung kann später natürlich wieder gelöst werden, um die für die Gliedmassen lebenswichtige Blutzirkulation wieder herzustellen.

Der erfindungsgemässe ortsdefiniert aufklebbare Gefässverschluss hat somit speziell auch in Notfällen Vorteile gegenüber den üblicherweise eingesetzten und oft improvisierten Stauverbänden, da er nämlich immer auch hygienischen Aspekten genügt und ohne weiteres als steriler Einweg- oder Verbrauchsgegenstand konzipiert werden kann.

### Bezugsziffernliste:

- 1: Gefässverschluss
- 2: Trägerschicht
- 3: (Medizinische) Hautkleberschicht
- 4: (Technische) Kleberschicht
- 5: Verschlussschicht
- 6: Druckkörper
- 7: Halteband
- 8: Nadel oder Kanüle

- U: Unterseite der Trägerschicht

## Patentansprüche

1. Aufklebbarer Gefässverschluss (1) zum Verschliessen eines eine Punktionsöffnung aufweisenden Blutgefässes oder einer unfallbedingten Gefässeröffnung, mit
- einer Trägerschicht (2),
- einem auf der Trägerschicht (2) angebrachten Verschlusselement mit einem Druckkörper (6), wobei der Druckkörper (6) durch eine Öffnung der Trägerschicht (2) ragt,
- einer auf der Unterseite U der Trägerschicht (2) angebrachten Hautkleberschicht (3) ; wobei
- das Verschlüsselement nebst dem Druckkörper (6) eine Verschlussschicht (5) aufweist;
und
- zwischen der Trägerschicht (2) und der Verschlussschicht (5) eine Kleberschicht (4) vorhanden ist;
**dadurch gekennzeichnet, dass**
- die Trägerschicht (2) folienartig ausgebildet ist,
- und daß die Verschlussschicht (5) und der Druckkörper (6) einstückig ausgebildet sind.

2. Aufklebbarer Gefässverschluss nach Patentanspruch 1, **dadurch gekennzeichnet, dass** die Trägerschicht (2) stabilisierende Eigenschaften aufweist und weniger dehnbar ist als das Verschlusselement.

3. Aufklebbarer Gefässverschluss nach Patentanspruch 1 oder 2, **dadurch gekennzeichnet, dass** die Trägerschicht (2) flüssigkeitsabsorbierend ist.

4. Aufklebbarer Gefässverschluss nach einem der Patentansprüche 1 bis 3, **dadurch gekennzeichnet, dass** die Kleberschicht (4) keine hautkleberspezifischen Eigenschaften aufzuweisen braucht.

5. Aufklebbarer Gefässverschluss nach einem der Patentansprüche 1 bis 4, **dadurch gekennzeichnet, dass** die Kleberschicht (4) einen Trägerfilm aufweist der beidseitig mit Kleber versehen ist.

6. Aufklebbarer Gefässverschluss nach einem der Patentansprüche 1 bis 5, **dadurch gekennzeichnet, dass** die Hautkleberschicht Wirkstoffe mit antiallergischen Eigenschaften enthält.

7. Aufklebbarer Gefässverschluss nach einem der Patentansprüche 1 bis 6, **dadurch gekennzeichnet, dass** das Verschlusselement aus Naturkautschuk, Kunstkautschuk, Gummi, Latex, Silikon, Hydrogel, polymerem Kunststoff oder einer Kombination dieser Materialien besteht.

8. Aufklebbarer Gefässverschluss nach einem der Patentansprüche 1 bis 7, **dadurch gekennzeichnet, dass** das Verschlusselement vorzugsweise aus kleberschichtseitig plasma, corona-, nasschemisch- oder sonstwie behandeltem Silikon ist.

9. Aufklebbarer Gefässverschluss nach einem der Patentansprüche 1 bis 8, **dadurch gekennzeichnet, dass** die Trägerschicht (2), die Verschlussschicht (5), die Kleberschicht (4) und die Hautkleberschicht (3) transparent oder annähernd transparent sind.

10. Aufklebbarer Gefässverschluss nach einem der Patentansprüche 1 bis 9, **dadurch gekennzeichnet, dass** die Hautkleberschicht (3) von einer abziehbaren Schutzfolie bedeckt ist.

11. Aufklebbarer Gefässverschluss nach einem der Patentansprüche 1 bis 10, **dadurch gekennzeichnet, dass** Haltebänder (7) in einer für einen Stauverband geeigneten Länge vorhanden sind.

12. Aufklebbarer Gefässverschluss nach Patentanspruch 11, **dadurch gekennzeichnet, dass** die Haltebänder (7) wasserdampfdurchlässig sind.

## Claims

1. Adhesive vessel closure (1) for closing a punctured blood vessel or an accident-related vessel hole, comprising
- a support layer (2),
- a closing element provided with a pressure member (6), attached to said support layer (2), wherein said pressure member (6) projects through an opening in the support layer (2),
- a skin adhesive layer (3) mounted on the bottom face U of the support layer (2); wherein
- in addition to the pressure member (6), the closing element is provided with a closing layer (5)
and
- an adhesive layer (4) is provided between the support layer (2) and the closing layer (5)
**characterised in that**
- the support layer (2) is embodied in a film-type manner and that the closing layer (5) and the pressure member (6) are embodied monolithically.

2. The adhesive vessel closure according to claim 1, **characterised in that** the support layer (2) exhibits stabilising properties and is less expandable than the closing element.

3. The adhesive vessel closure according to claim 1 or 2, **characterised in that** the support layer (2) is liquid-absorbing.

4. The adhesive vessel closure according to any one of claims 1 to 3, **characterised in that** the adhesive layer (4) does not need to have any skin-adhesive-specific properties.

5. The adhesive vessel closure according to any one of claims 1 to 4, **characterised in that** the adhesive layer (4) comprises a supporting film which is provided with adhesive on both sides.

6. The adhesive vessel closure according to any one of claims 1 to 5, **characterised in that** the skin-adhesive layer contains active substances having anti-allergic properties.

7. The adhesive vessel closure according to any one of claims 1 to 6, **characterised in that** the closing element is made of natural rubber, synthetic rubber, vulcanised rubber, latex, silicone, hydrogel, polymeric plastic or a combination of these materials.

8. The adhesive vessel closure according to any one of claims 1 to 7, **characterised in that** the closing element is preferably made of silicone which has undergone plasma, corona, wet-chemical treatment or has been treated in some other way on the adhesive layer side.

9. The adhesive vessel closure according to any one of claims 1 to 8, **characterised in that** the support layer (2), the closing layer (5), the adhesive layer (4) and the skin adhesive layer (3) are transparent or approximately transparent.

10. The adhesive vessel closure according to any one of claims 1 to 9, **characterised in that** the skin-adhesive layer (3) is covered with a peelable protective film.

11. The adhesive vessel closure according to any one of claims 1 to 10, **characterised in that** retaining strips (7) are provided in a length suitable for a pressure dressing.

12. The adhesive vessel closure according to claim 11, **characterised in that** the retaining strips (7) are permeable to water vapour.

## Revendications

1. Fermeture de vaisseau collante (1) pour fermer un vaisseau sanguin comportant un orifice de ponction ou un orifice de vaisseau dû à une blessure, avec
- une couche support (2),
- un élément de fermeture monté sur la couche support (2), avec un corps de pression (6), le corps de pression (6) saillant à travers un orifice de la couche support (2),
- une couche d'adhésif cutané (3) appliquée sur la face inférieure U de la couche support (2), l'élément de fermeture, comportant, hormis le corps de pression (6) une couche de fermeture (5),
- une couche d'adhésif (4) étant présente entre la couche support (2) et la couche de fermeture (5),
**caractérisée en ce que**
- la couche support (2) est conçue sous la forme d'un film et **en ce que** la couche de fermeture (5) et le corps de pression (6) sont conçus en monobloc.

2. Fermeture de vaisseau collante selon la revendication 1, **caractérisée en ce que** la couche support (2) a des propriétés stabilisatrices et est moins extensible que l'élément de fermeture.

3. Fermeture de vaisseau collante selon la revendication 1 ou 2, **caractérisée en ce que** la couche support (2) absorbe les liquides.

4. Fermeture de vaisseau collante selon l'une quelconque des revendications 1 à 3, **caractérisée en ce que** la couche adhésive (4) n'a pas besoin de présenter des propriétés spécifiques à un adhésif cutané.

5. Fermeture de vaisseau collante selon l'une quelconque des revendications 1 à 4, **caractérisée en ce que** la couche adhésive (4) comporte un film support, muni d'adhésif sur ses deux faces.

6. Fermeture de vaisseau collante selon l'une quelconque des revendications 1 à 5, **caractérisée en ce que** la couche d'adhésif cutané contient des principes actifs avec des propriétés antiallergiques.

7. Fermeture de vaisseau collante selon l'une quelconque des revendications 1 à 6, **caractérisée en ce que** l'élément de fermeture est en caoutchouc naturel, en caoutchouc synthétique, en gomme, en latex, en silicone, en hydrogel, en matière plastique polymère ou en une combinaison desdites matières.

8. Fermeture de vaisseau collante selon l'une quelconque des revendications 1 à 7, **caractérisée en ce que** l'élément de fermeture est traité côté couche d'adhésif de préférence au plasma, corona, par traitement chimique humide ou par un autre traitement.

9. Fermeture de vaisseau collante selon l'une quelconque des revendications 1 à 8, **caractérisée en ce que** la couche support (2), la couche de fermeture (5), la couche d'adhésif (4) et la couche d'adhésif cutané (3) sont transparentes ou presque transparentes.

10. Fermeture de vaisseau collante selon l'une quelconque des revendications 1 à 9, **caractérisée en ce que** la couche d'adhésif cutané (3) est recouverte d'un film de protection pelliculable.

11. Fermeture de vaisseau collante selon l'une quelconque des revendications 1 à 10, **caractérisée en ce que** des bandes de maintien (7) sont présentes, dans une longueur adaptée pour un bandage de compression.

12. Fermeture de vaisseau collante selon la revendication 11, **caractérisée en ce que** les bandes de maintien (7) sont perméables à la vapeur d'eau.
